# EUROPEAN PATENT APPLICATION

(11) **EP 2 990 976 A1**
(43) Date of publication of application: **02.03.2016**
(21) Application number: 14182700.6
(22) Date of filing: 28.08.2014
(51) Int. Cl.: G06F 19/18, G06F 19/22

(54) **Functional annotation of sequence**

(71) Applicant: Siemens Aktiengesellschaft, 80333 München (DE)
(72) Inventor: Kremer, Andreas, 54292 Trier (DE); Posch, Andreas Emanuel, 1090 Wien (AT); Winkler, Erich, 2392 Grub (AT)
(74) Representative: Maier, Daniel Oliver

(57) **Abstract**

Present application relates to a computer-implemented method for Functional Annotation of biological sequences comprising steps of:
- Loading all common representations of variations of a biological sequence of interest - if applicable per chromosome - with the information about the position (POS), reference (REF) and alternative (ALT) allele as well as - if applicable - genotype in a working space of a memory of a computer;
- Loading for each variation a predetermined number (N_{window}) of elements of the sequence upstream to the position (POS) from the human reference genome;
- Determining the lowest possible variant position (POS) and variant count for each variation and realigning annotations and defining the constituent unambiguous minimal variants;
- Describing personal variants based on their constituent haplotypes/unambiguous minimal variants/personal variants;
- Annotating personal variants based on their constituent haplotypes / unambiguous minimal variants / personal variants.

The invention enables harmonization of clinical annotation databases and variant caller output (e.g. vcf-files) by unambiguous minimal variant description using the lowest possible variant position and variant count.

## Description

Next-generation genomic sequencing (NGS) generally produces short reads or short read pairs, meaning short sequences of <∼200 bases. To compare the DNA of the sequenced sample to its reference sequence, it is necessary to find the corresponding part of that sequence for each read in the sequencing data.

This is called aligning or mapping the reads against the reference sequence. Once this is done, variations within the sample can be identified (variant calling).
The process of attaching biological information to sequences is called annotation. If the sequence describes a genome, it consists of two main steps:
- identifying portions of the genome that are different from the reference genome (i.e. personal variants) in coding and non-coding (e.g. regulatory) regions.
- attaching biological information (e.g. disease relevant information) to these variants.

Genome annotation is an active area of investigation and involves a number of different organizations in the life science community which publish the results of their efforts in publicly available biological databases accessible via the web and other electronic means like proprietary databases.

These results are particularly important for personalized medicine, when the annotation describes the relevance of certain genome sequence variants for diseases and suitable treatment measures.

Currently, no standard nomenclature for unambiguous description of sequence variants exists. While the Human Genome Variation Society suggests guidelines for description of sequence variants, these are not implemented across all NGS pipelines especially for descriptions of complex sequence variants such as deletions, insertions (including duplications) or the combination of both.

As a result, unambiguous annotation of personal sequence variants with information from clinical annotation databases still commonly involves manual curation of results.

Similarly, no common standard for identification of unambiguous personal variants from next-generation sequencing exists. So, different variant callers may report sequence variations differently in the vcf (variant call format) output file as shown in tables 1 and 2. Thereby, standardized and automated functional annotation of sequence variants with clinically relevant information is inhibited.

Table 1. a) Reference genome sequence and alternative sequence with deletion of position 2 and 3.
b) Examples of different versions of variant caller outputs in vcf-format like representation.
   a) 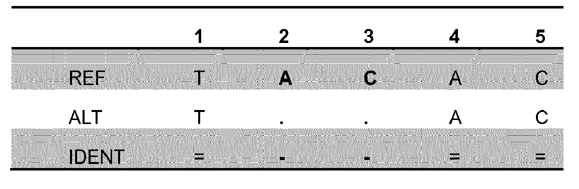
   b)

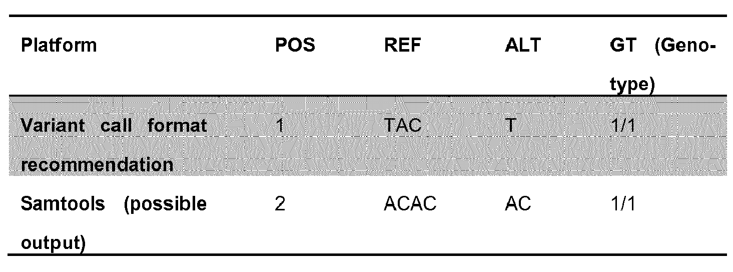

As described in Danecek et al. "The variant call format and VCFtools"; The variant call format and VCFtools Bioinformatics (2011) 27 (15): 2156-2158;

POS, refers to a 1-based position of the start of the variant REF, refers to the reference allele
ALT, refers to alternate non-reference alleles
GT, refers to an individual's genotype with regard to a particular gene of interest and, in polyploid individuals, it refers to what combination of alleles the individual carries (homozygous or heterozygous)

Table 2. a) Reference genome sequence REF and alternative sequence ALT with substitutions at position 2 and 3. b) Examples of different versions of variant caller outputs in vcf-format like representation.
a) 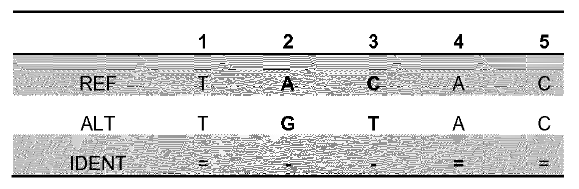
b)

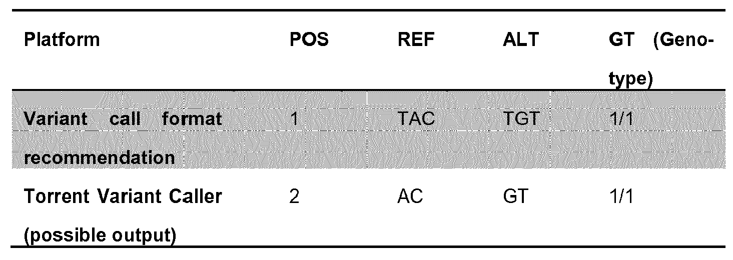

As shown in the tables, different variant callers and clinical annotation databases exhibit significant variations in the representation of sequence variants.

Therefore, existing approaches for functional annotation of variants commonly comprise two steps.

In a first step, any clinical annotation overlapping the variant position is reported, e.g. by intersecting vcf-files providing personal variants with bed-files providing clinical annotations (for example Genome Trax™ (Biobase AG, D) is a well-known collection including several variation and mutation databases such as COSMIC or HGMD).

In a second step, such derived clinical annotations will be manually sorted and prioritized for identical variants, i.e. identical positions and nucleotide changes/ insertions/ deletions; similar variants, i.e. identical positions but different nucleotide changes/insertions/deletions or only partially identical overlaps of variants in the vcf-file with clinical annotations.

With respect to the example shown in Table 1, this two-step manual approach will fail reporting relevant overlaps in case the variant caller reports a deletion of the nucleotides A and C at position 2 and 3 and the clinical annotation source provides effect annotation for deletion of A and C at position 4 and 5.

However, due to the repetitive character of the sequence, deletion of nucleotides at position 2 and 3 equals the deletion of nucleotides at position 4 and 5.

As a result, unambiguous annotation of sequence variants with information from clinical knowledge databases still remains a time-consuming task involving several manual curation steps.

It is object of the present invention to simplify the curation of annotation results and facilitate the (automatic) analysis and annotation of unambiguous personal variants.

According to the invention this problem is solved with the method of claim 1.

Preferred embodiments of the invention are shown in the dependent claims.

The invention enables harmonization of clinical annotation databases and variant caller output (e.g. vcf-files) by unambiguous minimal variant description using the lowest possible variant position and variant count. Thereby it facilitates automated functional annotation of personal variants for clinical decision support based on unambiguous minimal variants as unique keys. Moreover, the introduction of UMVs for indivisible representation of sequence variants targets at subsequent impact evaluation and separation of driver mutations from passenger mutations for complex variants.

Non limiting examples for carrying out the invention are described with reference to the drawings.
Fig. 1 shows a flowchart of the computer-implemented method for Functional Annotation of biological sequences, and
Fig. 2 illustrates an example reference genome sequence and alternative sequence.

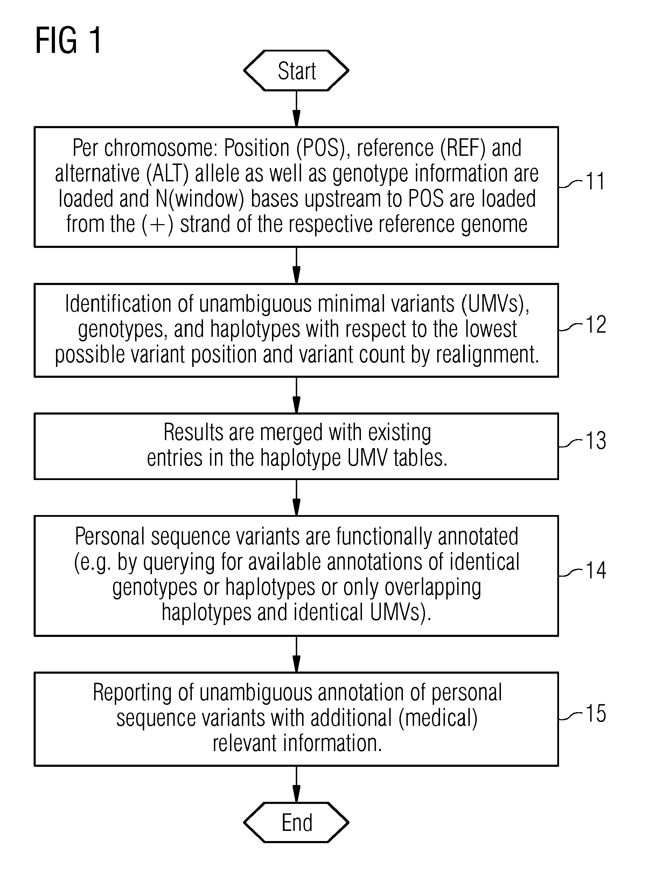

The flowchart shown in Fig. 1 represents a workflow example to derive unambiguous annotation of sequence variants with medical relevant information.

Personal/Annotated Variants refer to one or - for genotypes-two Haplotypes. Each Haplotype refers to at least one unambiguous minimal variants (and each unambiguous minimal variants is part of at least one Haplotype).

The workflow starts in a first step 11 with the loading of position POS, reference REF and alternative ALT allele as well as genotype information and N(window) bases upstream to position POS from the plus strand + of the respective reference genome per chromosome.

A second step 12 comprises the identification of unambiguous minimal variants, genotypes, and haplotypes with respect to the lowest possible variant position and variant count by realignment.

In a third step 13 results are merged with existing entries in the haplotype and unambiguous minimal variant tables.

In a fourth step 14, personal sequence variants are functionally annotated (e.g. by querying for available annotations of identical genotypes or haplotypes or only overlapping haplotypes and identical unambiguous minimal variants).

The workflow ends with a fifth step 15, the reporting of unambiguous annotation of personal sequence variants with additional (medical) relevant information.

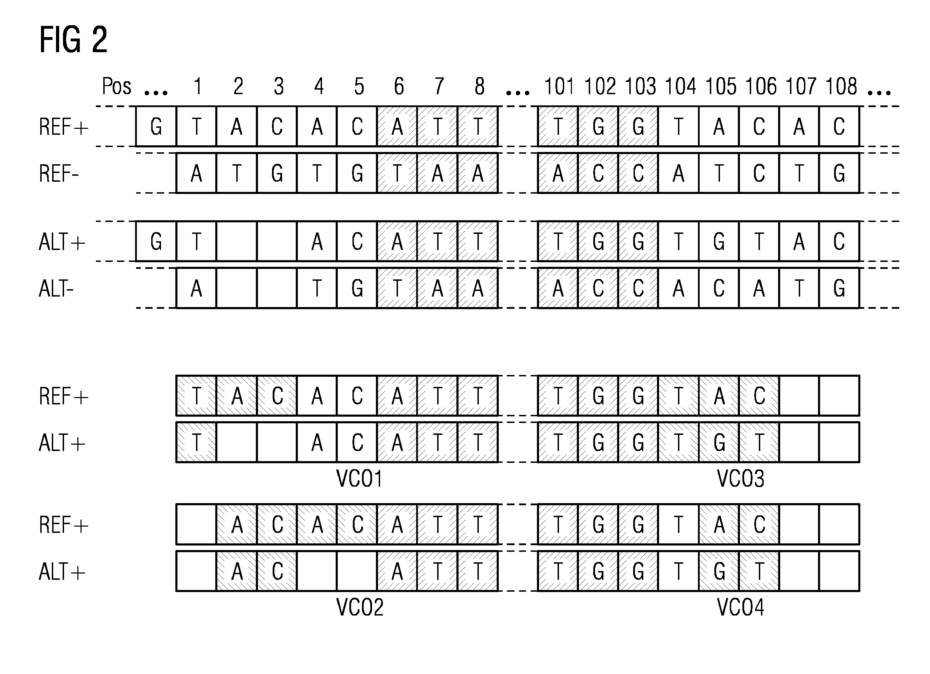

Fig 2 shows plus strand and minus strand of an example reference genome sequence REF+, REF- and alternative sequence ALT+, ALT-, with deletion of position 2 and 3 and with substitutions at position 105 and 106.

Due to the lack of common standard for identification of personal variants from next-generation sequencing different variant callers - e.g. samtools or Torrent Variant Caller - and clinical databases may report sequence variations differently.

Four examples of different versions of variant caller outputs (VCO) as illustrated in Table 1 and 2 are visualized for the plus strand of reference genome sequence.

So, in example 1 VCOl, variant 1 will be located at position 1 with a length of 3 bases (TAC vs. T..) and in example VCO3 variant 2 will have the position 104 also with a length of 3 bases (TAC vs. TGT).

In Example 2 VCO2, variant 1 would be located at position 2 with a length of 4 bases. (ACAC vs...AC) and in VCO4 variant 2 would have the position 105 with a length of 2 bases (AC vs. GT)

According to the invention, for each variant the lowest possible position and minimal length is determined.

Therefore the first variant will be locate at position 2 with a length of 2 bases (AC vs. ..) and the second variant will be located at position 105 with a length of 2 bases (AC vs. GT).

This unambiguous description of variants enables the automated procession of variants and annotation information and improves the usability of this information especially for clinical decision support systems.

## Claims

1. Computer-implemented method for Functional Annotation of biological sequences comprising steps of:
- Loading all common representations of variations of a biological sequence of interest - if applicable per chromosome - with the information about the position (POS), reference (REF) and alternative (ALT) allele as well as - if applicable - genotype in a working space of a memory of a computer;
- Loading for each variation a predetermined number (N_{window}) of elements of the sequence upstream to the position (POS) from the human reference genome (11);
- Determining the lowest possible variant position (POS) and variant count for each variation and realigning annotations and defining the constituent unambiguous minimal variants;
- Describing personal variants based on their constituent haplotypes / unambiguous minimal variants / personal variants;
- Annotating personal variants based on their constituent haplotypes / unambiguous minimal variants / personal variants.

2. The computer-implemented method of claim 1 where the information is derived from clinical databases further comprising steps of:
- Proofing if reference (REF) and alternative (ALT) allele are described for the (+) strand, and if not,
- Transforming reference (REF) and alternative (ALT) allele to (+) strand representation by reverse complementation.

3. The computer-implemented method of claim 1 where personal variants of genome sequences are annotated, further comprising steps of:
- Merging of genotypes and removing of duplicates from the personal variants described in the vcf-file.
